(19) Europäisches Patentamt
European Patent Office
Office européen des brevets

(11) EP 3 299 085 A1

(12) EUROPEAN PATENT APPLICATION

(43) Date of publication:
**28.03.2018 Bulletin 2018/13**

(51) Int Cl.:
***B01J 19/00*** *(2006.01)*

(21) Application number: **16190641.7**

(22) Date of filing: **26.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Enzypep B.V.**
**6167 RD Geleen (NL)**

(72) Inventors:
- **Quaedflieg, Peter Jan Leonard Mario**
  **6167 RD Geleen (NL)**
- **Nuijens, Timo**
  **6167 RD Geleen (NL)**
- **Lax, Edmund Rodney**
  **6167 RB Geleen (NL)**
- **Toplak, Ana**
  **6167 RD Geleen (NL)**

(74) Representative: **V.O.**
**P.O. Box 87930**
**Carnegieplein 5**
**2508 DH Den Haag (NL)**

(54) **PEPTIDE COUPLING MOLECULE**

(57) The invention relates to a peptide coupling molecule represented by the formula

$$L\{-\text{PeptideY-Q}\}_x$$

wherein
x is an integer having a value of at least 2;
L is a linker moiety;
each PeptideY independently represents a peptide chain comprising at least four amino acid units, each PeptideY having a terminal carbonyl group (C=O) and each Q independently represents a functional group forming an ester or thioester functionality with the terminal carbonyl group of the PeptideY to which Q is bound ; and
at least two PeptideY's of the peptide coupling molecule are different from each other.

EP 3 299 085 A1

## Description

The invention relates to a peptide coupling molecule, which may be used in the synthesis of peptide conjugates. The invention further relates to a method for synthesizing a conjugate. The invention further relates to a peptide conjugate. The invention further relates to a peptide conjugate for use as a medicament. The invention further relates to a peptide coupling reagent product.

Peptides have many applications, for instance as a pharmaceutical, food ingredient, feed ingredient or cosmetic ingredient.

A peptide may be used as such, *e.g.* as a pharmaceutically active agent, or be part of a conjugate further comprising one or more other moieties, *e.g.* another peptide, a protein, a carbohydrate or a lipid. Thus, several functions of the individual moieties can be combined in a single molecule. The provision of, *e.g.,* a conjugate of a pharmaceutically active peptide and a further moiety can be useful to alter an absorption, distribution, metabolism or excretion (ADME) characteristic, *e.g.* increase solubility in blood plasma or increase half-life of the pharmaceutically active peptide. Another reason to couple another moiety to the peptide is to provide it with another pharmaceutically active agent. Further, there is a need for conjugates of peptides and a label, *e.g.* for imaging or analytical purposes, *e.g.* a fluorescent, phosphorescent, chromogenic or radioactive group. A peptide conjugate containing a chelation agent or toxin is also useful.

Chemo-enzymatic coupling methods are advantageous over chemical coupling methods, amongst others, because they are generally more selective and can be carried out without racemisation. For instance, methods are known to selectively couple peptides by coupling (a) an (oligo)peptide C-terminal ester or thioester and (b) an (oligo)peptide nucleophile having an *N*-terminally unprotected amine, see *e.g.* US 5,403,737 or WO 2016/056913. However, such methodology only allows coupling the C-terminus of one peptide with the N-terminus of the other. It would be desirable to couple a peptide via its N-terminus with another peptide via the N-terminus of said other peptide or with another compound having an amine functionality. After all, the position via which coupling is accomplished can affect a property of a peptide, such as its biological activity or an ADME characteristic. Further, the C-terminus of a peptide may be shielded by the remainder of the peptide, thereby hampering its participation in the coupling reaction. Such shielding is in particular prominent if one or both the compounds have a tertiary structure.

It is an object of the invention to provide a way to allow coupling a peptide via its N-terminus with another compound having an amine functionality, in particular with another peptide via the N-terminus of said other peptide.

It is in particular an object of the invention to provide a coupling method of a peptide via its N-terminus with another compound, in particular an other peptide, having an amine functionality, allowing selective coupling without needing side-chain protection.

It is a further object of the invention to provide the coupling of a peptide with a protein via the N-termini of the peptide and the protein.

One or more other objects that may be subject of the invention follow from the description below.

It has now been found possible to couple a peptide via its N-terminus with another compound having an amine functionality, in particular with another peptide via the N-terminus of said other peptide (wich peptide may be a protein via the N-terminus of said protein). This is accomplished with a specific chemo-enzymatic method, wherein a specific peptide coupling molecule is used via which said peptide and said other compound(s) are linked.

Accordingly, the present invention relates to a peptide coupling molecule represented by the formula

$$L\{\text{- PeptideY-Q}\}_x$$

wherein

x is an integer having a value of at least 2;
L is a linker moiety;
each PeptideY independently represents a peptide chain comprising at least four amino acid units, each PeptideY having a terminal carbonyl group (C=O)
each Q independently represents a functional group forming an ester or thioester functionality with the terminal carbonyl group (C=O) of the PeptideY to which Q is bound; and
at least two PeptideY 's of the peptide coupling molecule are different from each other.

Further, the invention relates to a method for synthesizing a conjugate comprising enzymatically coupling a first nucleophile having an N-terminally unprotected amine (preferably a first peptide having an N-terminally unprotected amine) to a first ester or thioester functionality Q-(C=O) of a peptide coupling molecule according to the invention, which coupling is catalysed by a first ligase and enzymatically coupling a second nucleophile, having an N-terminally unprotected amine (preferably a second peptide, different from the first peptide), having an *N*-terminally unprotected amine - to a second ester or thioester functionality Q-(C=O) of said peptide coupling molecule, which coupling is catalysed by a

second ligase.

The invention further relates to a peptide conjugate represented by the formula

$$L\{\text{-PeptideY-(C=O)-PeptideZ}\}_x$$

wherein

x, PeptideY and L are as defined for the peptide coupling molecule according to the invention , and wherein

each PeptideZ is a peptide bound to -(C=O)- PeptideY via its N-terminal amino acid residue.

The invention further relates to a peptide conjugate according to the invention , comprising at least one pharmaceutically active PeptideY for use as a medicament.

The invention further relates to a pharmaceutical composition comprising a a peptide conjugate according to the invention, said conjugate comprising at least one pharmaceutically active PeptideY and a pharmaceutically acceptable excipient.

The invention further relates to a peptide coupling reagent product, comprising a peptide coupling molecule according to the invention, a first ligase and a second ligase.

The peptide coupling molecule according to the invention respectively the conjugate according to the invention comprises two or more ' PeptideY', moieties. At least two of these are different from each other, *i.e.* have a different amino acid sequence. Generally, they have at least one difference in the four amino acid units at the C-terminal end of the peptide (P1-P2-P3-P4), preferably in at least one of the first (P1), second (P2) or fourth (P4) amino acid counted from the C-terminus. This allows selective enzymatic coupling of a first nucleophile having an N-terminally unprotected amine to a first ester or thioester functionality Q-(C=O) of the peptide coupling molecule, by using a first ligase with a high selectivity towards coupling the first C-terminal ester or thioester to said first nucleophile (compared to coupling said first nucleophile to the second C-terminal ester or thioester) and/or by using a second ligase with a high selectivity towards coupling the second C-terminal ester or thioester to said second nucleophile (compared to coupling said second nucleophile to the first C-terminal ester or thioester). One or more further nucleophiles may be enzymatically coupled to the peptide linker molecule, using one or more further ligases.

For the purpose of this invention, with "synthesis over hydrolysis ratio" (S/H ratio) is meant the amount of enzymatically synthesised peptide product divided by the amount of the C-terminal ester or thioester of which the ester or thioester group has been hydrolysed.

The value of the S/H ratio of a ligase of the invention depends on various factors, for instance the nature of the substrates and reaction conditions (*e.g.* temperature, pH, concentration of the peptide nucleophiles and peptide coupling molecule, ligase concentration).

Further, following a method of the invention, the peptide conjugation product is easy to purify from the reaction mixture because only little hydrolytic byproducts are formed.

The term "or" as used herein is defined as "and/or" unless it is specified otherwise or it follows from the context that it means 'either ....or...' .

The term "a" or "an" as used herein is defined as "at least one" unless it is specified otherwise or it follows from the context that it should refer to the singular only.

When referring to a noun (*e.g.* a compound, an additive, etc.) in the singular, the plural is meant to be included, unless it follows from the context that it should refer to the singular only.

In the context of this application, the term "about" means in particular a deviation of 10 % or less from the given value, more in particular 5 % or less, even more in particular 3 % or less.

The term "essential(ly)" or "(at least) substantial(ly)" is generally used herein to indicate that it has the general character or function of that which is specified. When referring to a quantifiable feature, this term is in particular used to indicate that it is at least 75 %, more in particular more than 90 %, even more in particular more than 98 % of the maximum of that feature. The term "essentially free" is generally used herein to indicate that a substance is not present (below the detection limit achievable with analytical methodology as available on the effective filing date) or present in such a low amount that it does not significantly affect the property of the product that is essentially free of said substance. In practice, in quantitative terms, a product is usually considered essentially free of a substance, if the content of the substance is 0 - 0.1 wt.%, in particular 0 - 0.01 wt.%, more in particular 0 - 0.005 wt.%, based on total weight of the product in which it is present.

When referring to a compound of which stereoisomers exist, the compound may be any of such stereoisomers or a mixture thereof. Thus, when referred to, *e.g.,* an amino acid of which enantiomers exist, the amino acid may be the L-enantiomer, the D-enantiomer or a mixture thereof. In case a natural stereoisomer exists, the compound is preferably a natural stereoisomer.

The term 'pH' is used herein for the apparent pH, *i.e.* the pH as measured with a standard, calibrated pH electrode.

For the purpose of this invention, with "peptides" is meant any chain composed of two or more amino acids. Thus, peptides are generally amides at least conceptually composed of two or more amino carboxylic acid molecules

(*i.e.* amino acids) by formation of a covalent bond from the carbonyl carbon of one to the nitrogen atom of another with formal loss of water. The term 'peptide' includes oligopeptides and polypeptides. The term 'peptide' is usually applied to structures formed from alpha-amino acids, although a peptide may comprise other amino acids, such as one or more beta-amino acids and/or one or more gamma-amino acids. A peptide may be linear, branched or cyclic. A peptide can have a single chain composed of two or more amino acids or a peptide can have a plurality of chains (i.e. a chimeric peptide). In the case a peptide is composed of two or more chains, each chain generally is composed of three or more amino acid molecules.

The amino acid sequence of a peptide is referred to as the primary structure.

In an embodiment, the peptide is essentially free of a secondary structure and essentially free of a tertiary structure.

In a further embodiment, the peptide has a secondary structure. Secondary structures are generally highly regular local sub-structures, such as alpha-helices and beta-sheets (or beta-strands), by interactions between the individual amino acids and the peptide backbone.

In an embodiment, the peptide (which may be a chimeric peptide) has a tertiary structure. Tertiary structures are generally formed by multiple interactions, among others hydrogen bonding, hydrophobic interactions, van der Waals interactions, ionic interactions and disulphide bonds. The secondary structure can also contribute to the tertiary structure. The tertiary structure provides a three-dimensional shape (which is essentially fixed in a stable environment, such as in the absence of a change in temperature and in the absence of a change in the medium wherein the peptide is present, etc.). As the skilled person knows, the tertiary structure is different from a random coil peptide chain lacking any fixed three-dimensional structure. Proteins, like insulin, albumin, antibodies, biotin, peptide-based receptor ligands, are examples of peptides having a tertiary structure.

Disulphide bonds (disulphide bridges) are typically bonds between two cysteine units (formed by oxidation). Thus, two amino acids in a same peptide chain (amino acid sequence) can be covalently bound, also if they are not adjacent amino acids in the amino acid sequence. Also, a disulphide bond between a first cysteine of a first peptide chain and a second cysteine of a second peptide chain, which may have the same or a different amino acid sequence, can be formed to form a peptide. Such peptide comprises more than one peptide chain. An example of a peptide composed of more than one peptide chain, wherein the different chains are bound via a disulphide bond is insulin.

In an embodiment, a peptide (to be) coupled to the peptide coupling molecule essentially consists of amino acid units. In a further embodiment, the peptide essentially consists of amino acid units and protective groups.

In an embodiment, a peptide to be coupled is a conjugate of a peptide chain of two or more amino acids and another residue, such as a carbohydrate or a lipid. These peptides are called glycopeptides and lipopeptides respectively. Lipids can *e.g.* be used to change solubility. Examples of suitable lipids, are C8-C24 saturated fatty acids and C8-C24 unsaturated fatty acids.

In a further embodiment, a peptide (to be) coupled to the peptide coupling molecule is a peptide modified with a synthetic hydrophilic polymer, such as a polyalkylene glycol. Particularly preferred polyalkylene glycols are polyethylene glycols. Such polymers can, *e.g.* be used to change solubility or half-life.

In a further embodiment, a peptide (to be) coupled to the peptide coupling molecule is a conjugate of a peptide and a polysialic acid.

A peptide (to be) coupled to the peptide coupling molecule may be a conjugate of a peptide and an imaging agent, a radio-therapeutic agent, a toxin or another non-peptidic agent, *e.g.* a chelating agent or a non-peptidic medicament.

In an embodiment, a first peptide (to be) bound to the peptide coupling molecule is a pharmaceutically active (peptide, *e.g.* an insulin receptor ligand , an imaging agent (*e.g.* chromogenic, fluorescent, phosphorescent, radioactive) or a radio-therapeutic agent (forming part of a peptide) and a second peptide (to be) bound to the peptide coupling molecule is a protein for target delivery of the first peptide to a specific site, *e.g.* to specific organ tissue. Well known examples of proteins, suitable for such purpose, are immunoglobulins or parts thereof, such as an antigen-binding fragment (Fab) of an immunoglobulin.

In an embodiment, at least one peptide forming part of a peptide conjugate according to the invention is a protein suitable to increase the half-life of another (pharmaceutically active) peptide forming part of the conjugate in a living organism, in particular the blood plasma half-life. Albumins are examples of proteins that can be coupled to another peptide (via the peptide coupling molecule) to increase the half-life of said other peptide.

Typically, a peptide - which term includes oligopeptides, proteins and chimeric peptides - comprises up to about 35 000 amino acid units, in particular 3-20 000 amino acid units, more in particular 4-5 000 amino acid units, preferably 5-1000 amino acid units. In a specifically preferred embodiment the peptide comprises 500 amino acid units or less, in particular 200 or less, more in particular 100 or less. In a specifically preferred embodiment, the peptide comprises at least 10 amino acid units, more specifically at least 15 amino acids, at least 25 amino acids or at least 40 amino acids.

With "oligopeptides" is meant within the context of the invention, a peptide composed of 2-200 amino acid units, in particular composed of 5-100 amino acid units, more in particular composed of 10-50 amino acid units.

For the purpose of this invention, with "peptide bond" is meant the amide bond between (i) either the alpha-amino terminus of one alpha-amino acid or the beta-amino acid terminus of one beta-amino acid and (ii) either the alpha-carboxyl terminus of one other alpha-amino acid or the beta-carboxyl terminus of one other beta-amino acid. Preferably, the peptide bond is between the alpha-amino terminus of one amino acid and the alpha-carboxyl terminus of another amino acid.

In the context of the invention with "amino acid side-chain" is meant any proteinogenic or non-proteinogenic amino acid side-chain.

Proteinogenic amino acids are the amino acids that are encoded by the genetic code. Proteinogenic amino acids include: alanine (Ala), valine (Val), leucine (Leu), isoleucine (Ile), serine (Ser), threonine (Thr), methionine (Met), cysteine (Cys), asparagine (Asn), glutamine (Gln), tyrosine (Tyr), tryptophan (Trp), glycine (Gly), aspartic acid (Asp), glutamic acid (Glu), histidine (His), lysine (Lys), arginine (Arg), proline (Pro) and phenylalanine (Phe). Selenocysteine (Sec, U) is an amino acid, of which the structure corresponds to cysteine, with the proviso that it contains a selenium instead of a sulphur atom. Proteinogenic amino acids are the L-stereoisomers of said amino acids (except for glycine, which does not have a stereo-isomeric form).

Non-proteinogenic amino acids may in particular be selected amongst D-amino acids, L- or D-phenylglycine, DOPA (3,4-dihydroxy-L-phenylalanine), beta-amino acids, 4-fluoro-phenylalanine, or $C^{a}$-alkylated amino acids.

The term "(thio)ester" is used herein as short-hand for the phrase " ester or thioester".

The term "mutated" or "mutation" as used herein regarding proteins or polypeptides - in particular enzymes - means that at least one amino acid in the wild-type or naturally occurring protein or polypeptide sequence has been replaced with a different amino acid, inserted into, appended to, or deleted from the sequence via mutagenesis of nucleic acids encoding these amino acids. Mutagenesis is a well-known method in the art, and includes, for example, site-directed mutagenesis by means of PCR or via oligonucleotide-mediated mutagenesis as described in Sambrook et al., Molecular Cloning-A Laboratory Manual, 2nd ed., Vol. 1-3 (1989). The term "mutated" or "mutation" as used herein regarding genes means that at least one nucleotide in the nucleic acid sequence of that gene or a regulatory sequence thereof, has been replaced with a different nucleotide, has been inserted into, has been appended to, or has been deleted from the sequence via mutagenesis, resulting in the transcription of a protein sequence with a qualitatively of quantitatively altered function or resulting in the knock-out of that gene.

In the present specification, a shorthand for denoting amino acid substitutions employs the single letter amino acid code of the amino acid that is substituted, followed by the number designating where in the protein amino acid sequence the substitution is made. This number is the amino acid position of the wild-type amino acid sequence. Thus for the mutated amino acid sequence it is the amino acid position corresponding to the position with that number in the wild type enzyme. Due to one or more other mutations at a lower position (additions, insertions, deletions, etc.) the actual position does not need to be the same. The skilled person will be able to determine the corresponding positions using a generally known alignment technique, such as NEEDLE. The number is followed by the single letter code of the amino acid that replaces the wild-type amino acid therein. For example, G166S denotes the substitution of glycine at the position corresponding to position 166 to serine. X is used to indicate any other proteinogenic amino acid than the amino acid to be substituted. For example, G166X denotes the substitution of glycine 166 to any other proteinogenic amino acid.

The term "ligase" is used herein for an enzyme having catalytic activity in the coupling of two peptides by catalysing the formation of a peptide bond by coupling the C-terminus of a first peptide and the N-terminus of another peptide.

As defined by Schechter and Berger, the active site residues in proteases, including ligases are composed of contiguous pockets termed subsites. Each subsite pocket binds to a corresponding residue in the peptide substrate sequence, referred to here as the sequence position. According to this definition, amino acid residues in the substrate sequence are consecutively numbered outward from the cleavage sites as...-P4-P3-P2-P1-P1'-P2'-P3'-P4'-...(the scissile bond is located between the P1 and P1' positions), while the subsites in the active site are correspondingly labelled as...-S4-S3-S2-S1-S1'-S2'-S3'-S4'-.(Schechter and Berger, Biochem Biophys Res Commun. 1967 Apr 20;27(2):157-62.)).

For the purpose of this invention, with "S1, S2, S3 and S4 pocket" is meant the amino acids of a protease (in particular a ligase) which interact with the amino acids of a peptide acyl donor. The C-terminal amino acid ($1^{st}$ amino acid; P1) of the acyl donor peptide interacts with the amino acids in the S1 pocket of the protease. The penultimate amino acid ($2^{nd}$ amino acid; P2) of the acyl donor peptide interacts with the amino acids in the S2 pocket of the protease, the third amino acid (P3) with the S3 and the fourth amino acid (P4) with the S4 pocket. The S1-S4 binding pockets of a protease are defined by several amino acids which can be distant in the primary structure of the protease, but are close in the three dimensional space. For the purpose of this invention, with S1' and S2' pockets are meant the amino acids of a protease which interact with the N-terminal amino acids of a peptide nucleophile. The N-terminal amino acid of the peptide nucleophile interacts with the amino acids in the S1' pocket of the protease. The N-terminal penultimate amino acid of the peptide nucleophile interacts with the amino acids in the S2' pocket of the protease. The S1' and S2' binding pockets of a protease are defined by several amino acids which can be distant in the primary structure of the

protease, but are close in the three dimensional space.

When an enzyme, such as a ligase, is mentioned with reference to an enzyme class (EC) between brackets, the enzyme class is a class wherein the enzyme is classified or may be classified, on the basis of the Enzyme Nomenclature provided by the Nomenclature Committee of the International Union of Biochemistry and Molecular Biology (NC-IUBMB), which nomenclature may be found at http://www.chem.qmul.ac.uk/iubmb/enzyme/. Other suitable enzymes that have not (yet) been classified in a specified class but may be classified as such, are meant to be included.

Homologues typically have an intended function in common with the peptide, such as a ligase, of which it is a homologue, such as being capable of catalyzing the same reaction, in particular an enzymatic coupling reaction of a method according to the invention.

Amino acid or nucleotide sequences are said to be homologous when exhibiting a certain level of similarity. Two sequences being homologous indicate a common evolutionary origin. Whether two homologous sequences are closely related or more distantly related is indicated by "percent identity" or "percent similarity", which is high or low respectively.

The terms "homology", "percent homology", "percent identity" or "percent similarity" are used interchangeably herein. For the purpose of this invention, it is defined here that in order to determine the percent identity of two amino acid sequences, the complete sequences are aligned for optimal comparison purposes. In order to optimize the alignment between the two sequences gaps may be introduced in any of the two sequences that are compared. Such alignment is carried out over the full length of the sequences being compared. Alternatively, the alignment may be carried out over a shorter length, for example over about 20, about 50, about 100 or more nucleic acids or amino acids. The percentage identity is the percentage of identical matches between the two sequences over the reported aligned region.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent identity between two amino acid sequences can be determined using the Needleman and Wunsch algorithm for the alignment of two sequences. (Needleman, S. B. and Wunsch, C. D. (1970) J. Mol. Biol. 48, 443-453). The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences, EBLOSUM62 is used for the substitution matrix. Other matrices can be specified. The optional parameters used for alignment of amino acid sequences are a gap-open penalty of 10 and a gap extension penalty of 0.5. The skilled person will appreciate that all these different parameters will yield slightly different results but that the overall percentage identity of two sequences is not significantly altered when using different algorithms.

The homology or identity between the two aligned sequences is calculated as follows: the number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labelled in the output of the program as "longest-identity". For purposes of the invention the level of identity (homology) between two sequences is calculated according to the definition of "longest-identity" as can be carried out by using the program NEEDLE.

The polypeptide sequences, in particular ligase sequences, can further be used as a "query sequence" to perform a search against sequence databases, for example to identify other family members or related sequences. Such searches can be performed using the BLAST programs. Software for performing BLAST analyses is publicly available through the National Center for Biotechnology Information (http://www.ncbi.nlm.nih.gov). BLASTP is used for amino acid sequences. The BLAST program uses as defaults:

- Cost to open gap: default = 11 for proteins
- Cost to extend gap: default = 1 for proteins
- Expect value: default = 10
- Wordsize: default = 28 for megablast/ 3 for proteins

Furthermore the degree of local identity (homology) between the amino acid sequence query and the retrieved homologous sequences is determined by the BLAST program. However only those sequence segments are compared that give a match above a certain threshold. Accordingly the program calculates the identity only for these matching segments. Therefore the identity calculated in this way is referred to as local identity.

The term "homologue" is used herein in particular for peptides, more in particular ligases, having a sequence identity of at least 50 %, preferably at least 60 %, more preferably at least 70 %, at least 80 %, at least 90 %, at least 95 %, at least 96 %, at least 97 %, at least 98 % or at least 99 % with the peptide with which the homologue peptide is

compared. Evidently, the sequence identity will be less than 100 %. The percentage of sequence identity will depend on the number of mutations and the length of the peptide with which the homologue is prepared. In 'longest identity' alignment deletions are not taken into account.

For the purpose of this invention, with "condensation" is meant the formation of a new amide bond between the C-terminal carboxylic function of a peptide (in particular a PeptideY) with the N-terminal amine function of a nucleophile, in particular another peptide.

The term "analogue" of a peptide is used in particular for peptides that are structural analogues and/or functional analogues of said peptide. Functional analogues have a same *in vivo* target (*e.g.* the same target receptor on a cell membrane); structural analogues have a high similarity in amino acid sequence. Functional analogues of a peptide may have a relatively low amino acid sequence identity, *e.g.* of about 50 % or less over the full amino acid sequence, yet at high sequence identity (and thus high structural similarity) with the peptide of which they are an analogue in a segment of the amino acid sequence, such as near the N-terminal part or near the C-terminal part. A structural analogue, in particular comprises an amino acid sequence that has at least 60 %, more in particular at least 70 %, preferably at least 80 %, more preferably at least 90 % sequence identity, more preferably at least 95 % sequence identity with the amino acid sequence of the peptide of which a peptide is an analogue.

For the purpose of clarity and a concise description features are described herein as part of the same or separate embodiments, however, it will be appreciated that the scope of the invention may include embodiments having combinations of all or some of the features described. Terms used herein that are not specifically defined herein are as defined in WO 2016/056913, or - if not defined therein - used in accordance with common general knowledge.

The term '*C*-terminal protection' is used herein to indicate that a *C*-terminal carboxylic group of a peptide is provided with a protective group, generally substantially protecting the carboxylic group from being coupled to an N-terminal amine group of a nucleophile, in particular another peptide. The *C*-terminal protective group may be a t-alkyl ester group for instance a t-butyl ester group, which is a commonly used protective group. The *C*-terminal protective group may also be a *C*-terminal carboxy-amide. A primary carboxy-amide is a commonly used protective group.

The term '*N*-terminal protection' is used herein to indicate that an *N*-terminal amine group of a peptide is provided with a protective group, generally at least substantially protecting the *N*-terminal amine group from being coupled to a *C*-terminal carboxylic group of another peptide or of the same peptide molecule. Suitable *N*- -protecting groups for terminal or side-chain N-protection are known to the person skilled in the art. Examples of suitable *N*-protecting groups include carbamate or acyl type protecting groups, for instance 'Cbz' (benzyloxycarbonyl), 'Boc' (tert-butyloxycarbonyl), 'For' (formyl), 'Fmoc' (9-fluorenylmethoxycarbonyl), 'PhAc' (phenacetyl) and 'Ac' (acetyl). The groups For, PhAc and Ac may be introduced and cleaved enzymatically using the enzymes Peptide Deformylase, PenG acylase or Acylase, respectively. Chemical cleavage methods are generally known in the art.

The ester or thioester functionalities Q- (C=O) of the peptide coupling molecule typically are activated (thio)esters, *i.e.* they contain a carboxy ester or carboxy thioester group that can take part in the enzymatic coupling reaction. Q may be depicted as 'RO' (for an ester) or 'RS' (for a thioester), wherein R represents an organic group. In particular, R can be selected from any (substituted or unsubstituted) alkyl or (substituted or unsubstituted) aryl (thio)ester.Typical examples of (thio)esters which can be used to take part in the enzymatic coupling reaction are methyl-, ethyl, propyl-, isopropyl-, phenyl-, benzyl-, 2,2,2-trichloroethyl-, 2,2,2-trifluoroethyl-, cyanomethyl- and carboxyamidomethyl-(thio)esters.

Preferably at least one Q, preferably each Q, - represents a structure with a formula $-OCX_2-Z$ or $-SCX_2-Z$, wherein each X independently represents a hydrogen atom, a halogen atom, a substituted or unsubstituted alkyl group (e.g. having 1-10 carbons, in particular 1-7 carbons) or a substituted or unsubstituted aryl group (e.g. having 6-12 carbons). and Z is selected from the group of $sp^3$-hybridized carbons comprising at least two substituents comprising a heteroatom directly attached to the $sp^3$-hybridized carbon and $sp^2$ hybridized carbons comprising one or two substituents comprising a heteroatom directly attached to the $sp^2$-hybridized carbon. Preferred Z groups are selected from the group consisting of carbamoyl groups; trifluoroalkyl groups, in particular trifluoromethyl;, trichloroalkyl groups, in particular trichloromethyl; cyanoalkyl groups, in particular cyanomethyl. Further details about ester or thioester functionalities Q- (C=O) comprising such a group Q , and their use, can e.g. be found in WO 2010/057961 and WO 2016/056913.

In a particularly preferred embodiment, at least one Z , preferably each Z, represents the formula

$$-C(=O)(NR^1R^2)$$

wherein

$R^1$ represents a hydrogen atom or a substituted or unsubstituted alkyl group or a substituted or unsubstituted aryl group; and

$R^2$ represents a hydrogen atom or a substituted or unsubstituted alkyl group or a substituted or unsubstituted aryl group or an amino acid or a peptide residue with a C-terminal carboxyamide or carboxylic acid functionality, optionally protected on the side-chain functionality of the amino acid residue or on one or more of the side-chain functionalities of the peptide

residue.

Good results have been obtained with carboxyamidomethyl-type esters represented by the formula -PeptideY-(C=O)-O-$CX^1X^2$-C(=O)N-$R^1R^2$. Herein, each $X^1$ and $X^2$ independently represents a hydrogen atom or an alkyl group. Particular good results have been achieved when both $X^1$ and $X^2$ are a hydrogen atom.

Herein $R^1$ represents a hydrogen atom or an alkyl group and $R^2$ represents a hydrogen atom or an alkyl group or an amino acid or a peptide residue with a *C*-terminal carboxyamide or carboxylic acid functionality, optionally protected on the side-chain functionality of the amino acid or on one or more of the side-chain functionalities of the amino acids.

Herein, each alkyl group may independently represent a (substituted or unsubstituted) C1-C7 alkyl group, preferably a (substituted or unsubstituted) linear C1-C6 alkyl group, more preferably a (substituted or unsubstituted) linear C1-C3 alkyl group, and most preferably a methyl group.

Good results have in particular been achieved in a method of the invention wherein both $R^1$ and $R^2$ of the carboxyamidomethyl-type ester represent a hydrogen atom or wherein $R^1$ represents a hydrogen atom and $R^2$ represents an amino acid or peptide residue with a *C*-terminal carboxyamide or carboxylic acid functionality, optionally protected on the side-chain functionality of the amino acid or on one or more of the side-chain functionalities of the amino acids.

The PeptideY moieties are usually non-cyclic and usually linear peptide chains. The number of amino acids - n - of a PeptideY is at least 4. A length of 4 amino acid units or more is advantageous when using it as a substrate in a method according to the invention, because ligases, in particular serine endoproteases, such as subtilisins or variants or homologues thereof, generally have four active pockets on the active ester side each one interacting with one of the last four amino acid units of a peptide chain (see above). A longer chain may be useful, *e.g.* to modify solubility properties of the peptide coupling molecule. Generally, the value of n will be 20 or less, in particular 10 or less, preferably 8 or less, 7 or less or 6 or less. If a PeptideY comprises six or more amino acid units, it may be branched, provided that the final four amino acid units at the C-terminal side form a linear chain.

In principle a PeptideY may represent any sequence of amino acid units, in particular any sequence of proteinogenic amino acid units, as long as the peptide linking molecule comprises at least two different PeptideY moieties. This is to facilitate selective coupling of the first nucleophile to the first (thio)ester functionality Q-(C=O) and selective coupling of the second nucleophile to the second (thio)ester functionality Q-(C=O).

Desired ligases for coupling a nucleophile to a PeptideY can be chosen dependent on the amino acid sequence of PeptideY, and in particular on the basis of the first two amino acid units starting from the C-terminal (thio)ester, i.e. the amino acid unit in the P1 position and the amino acid in the P2 position. The skilled person will be able to make such choices of PeptideY moieties and ligases on the basis of the information disclosed herein, in combination with common general knowledge, in combination with the contents of the documents cited herein, in particular WO 2016/056913 or PCT/NL2016/050501 (to Enzypep B.V. having an application date of 8 July 2016), optionally in combination with a limited amount of testing. The contents of these documents are incorporated herein by reference.

The PeptideY moieties of the peptide coupling molecule are bound to a linker moiety L. Good results have been obtained with a peptide coupling molecule wherein at least one, preferably all, PeptideY moieties are bound to L via an amide bond at the respective N-termini of the PeptideY moieties. However, if a PeptideY moiety has a chain length of five or more, in particular six or more, amino acid units, the bond may be at any amino acid unit starting from P5 from the C-terminal end of the PeptideY moiety. In such case, the bond may be between L and a side-chain amine group or another functional group of an amino acid unit of the PeptideY.

Advantageously, a PeptideY moiety is bound to L at the N-terminus of the PeptideY moiety. As mentioned above, good results have been achieved with a peptide coupling molecule wherein the bonds between the PeptideY moieties and L are amide bonds. However the N-terminal amine, a side-chain amine (at P5 or more remote from the C-terminus) or another functional group, *e.g.* an -SH or -OH (at P5 or more remote from the C-terminus) of a PeptideY may also form another type of bond, for instance a carbamate, a thiocarbamate, a urethane, an imine, an aminomethylene or a disulphide bond with the linker moiety L.

The linker moiety L generally is an organic moiety. Apart from possible heteroatoms at the bonds with the PeptideY, it may consist of carbon and hydrogen. However, in an embodiment it comprises one or more further heteroatoms, *e.g.* it may comprise one or more oxygens forming ether moieties. L may be a non-cyclic, linear or branched, structure or it may comprise a cyclic structure, *e.g.* a (hetero)cycloalkyl or (hetero)aryl structure. L may be fully saturated (no double or triple carbon carbon bonds) or comprise one or more unsaturated carbon carbon bonds. In principle there is no limit to the size of moiety L. L usually contains 100 atoms or less, preferably up to 50 atoms, in particular up to 20 atoms, more in particular up to 12. L usually contains 90 carbons or less, preferably 30 carbons or less, in particular up to 16 carbons , more in particular up to 10 carbons. Good results have been achieved with a peptide coupling molecule, wherein L is at least conceptually based on a saturated functionalised alkane, in particular wherein L is at least conceptually based on a polycarboxylic acid, preferably a dicarboxylic acid or tricarboxylic acid.

When L is at least conceptually based on a dicarboxylic acid, L may be a saturated structure represented by the formula $-(O{=}C)(CH_2)_n$ (C=O)- or an unsaturated structure represented by the formula $-(O{=}C)(C_nH_{2n-2p})$ (C=O)-. Herein n is an integer, preferably in the range of 1-14, in particular in the range of 2-8. Examples of groups represented

by this formula are oxaloyl (n=0), malonyl (n=1) succinyl (n=2), glutaryl (n=3), adipoyl (n=4) and sebacoyl (n=5). In said formula for the unsaturated structure 'p' is an integer indicating the number of unsaturations, usually in the range of 1-6, in particular 1, 2 or 3, and n is at least 2. Maleoyl (n=2, p=1) is an example of a moiety L having an unsaturated bond. Terephtaloyl is an example of an aromatic moiety at least conceptually based on teraphtalic acid.

When L is at least conceptually based on a tricarboxylic acid, L may be a saturated structure represented by the formula $(C_nH_{2n-1})$ $(C{=}O)_3$ or an unsaturated structure represented by the formula $(C_nH_{2n-1-2p})$ $(C{=}O)_3$. In said formula for the unsaturated structure 'p' is an integer indicating the number of unsaturations and n is at least 2. Citroyl (n=3) is an example of a saturated moiety L represented by the formula $(C_nH_{2n-1})$ $(C{=}O)_3$, trimesoyl is an example of an aromatic moiety providing three carbonyl groups.

In an embodiment, L is at least conceptually based on an amino acid having at least two carboxylic acid functionalities comprising two carbonyl groups forming an amide with an -NH- of a PeptideY . Aspartic acid and glutamic acid are examples of proteinogenic amino acids which may be used to provide a moiety L of a peptide linker molecule according to the invention. Diaminopimelic acid is an example of a non proteinogenic amino acid which may provide a moiety L.

In an embodiment, L comprises a peptide chain bound to each PeptideY.

Examples of such peptide chains are chains comprising a Lys and an Asp or Glu unit. Specific examples are peptide chains comprising a Lys-Asp, wherein the epsilon-amine of lysine has formed an amide with the beta-carboxylic acid of aspartic acid or Lys-Glu, wherein the epsilon-amine of lysine has formed an amide with the gamma-carboxylic acid of glutamic acid.

In an embodiment, L comprises two cysteine moieties which are bound via a disulphide bridge and which are either directly bound to a PeptideY moiety or part of a peptide chain bound to a PeptideY moiety.

In a particularly preferred embodiment, the peptide coupling molecule has two PeptideY moieties. Such peptide coupling molecule is preferably represented by the formula $Q_A[-(C{=}O)-(CR_AR_B)-(NH)-]_n-L-[(NH)-(CR_AR_B)-(C{=}O)-]_m-Q_B$.

Herein $Q_A$ and $Q_B$ are as defined for Q, preferably carboxyamidomethylesters;

n is an integer having a value of at least 4, in particular in the range of 4-20, more preferably in the range of 4-10, in particular 4, 5, 6 or 7;

m is in integer having a value of at least 4, in particular in the range of 4-20, preferably in the range of 4-10, more preferably, 4, 5, 6 or 7;

and each $R_A$ and each $R_B$ independently represent a hydrogen atom or an amino acid side-chain of a proteinogenic amino acid.

The peptide coupling molecule can be synthesized by providing building blocks for the peptide coupling molecule (a molecule providing L, *e.g.* a molecule containing two or more carboxylic acids, an anhydride, a molecule containing two or more isocyanate groups, a molecule containing two or more alkylchloroformate groups, a molecule containing two or more aldehyde groups, a molecule containing two or more C=C bonds, an amino acid or a peptide); PeptideY's and a molecule providing Q, typically an alcohol or thiol, and coupling the building blocks, based on reactions which may be known per se.

For instance, when L is provided by a dicarboxylic acid forming two amide bonds with the N-termini of two PeptideY-Q's in which both Q's are carboxyamidomethylesters (Cam-esters), then the peptide coupling molecule may be assembled by i) preparing a first side-chain protected PeptideY-Cam-ester via solid-phase peptide synthesis and cleaving it from the resin; ii) preparing a second side-chain protected PeptideY-Cam-ester on the solid phase, reacting it with an anhydride (e.g. succinic anhydride) and cleaving it from the resin; iii) coupling the free N-terminus of the first fragment with the free carboxylic acid moiety (i.e. the succinoyl moiety) of the second fragment using standard peptide coupling reagents such as N,N'-Dicyclohexylcarbodiimide (DCC) or (2-(1H-benzotriazol-1-yl)-1,1,3,3-tetramethyluronium / (E)-ethyl 2-cyano-2-(hydroxyimino)acetate(HBTU/Oxyma); iv) cleaving the side-chain protecting groups, giving the peptide coupling molecule based on two amide bonds between L and the two PeptideY-Q's.

For instance, when L is provided by a diisocyanate, forming two urethane moieties with the N-termini of two peptideY-Q's in which both Q's are Cam-esters, then the peptide coupling molecule may be assembled by i) preparing a first side-chain protected PeptideY-Cam-ester via solid-phase peptide synthesis and cleaving it from the resin; ii) preparing a second side-chain protected PeptideY-Cam-ester on the solid phase, reacting it with an excess of the diisocyanate, washing away the remaining diisocyanate and cleaving the resulting mono-isocyanate mono-urethane coupled side-chain protected PeptideY-Cam-ester from the resin; iii) coupling both PeptideY-Cam-esters by a reaction of the N-terminus of the first PeptideY-Cam-ester with the mono-isocyanate functionality of the second PeptideY-Cam-ester; cleaving the side-chain protecting groups, giving the peptide coupling molecule based on two urethane moieties.

For instance, when L is provided by a compound containing two chloroformate groups (-O(C=O)Cl groups) forming carbamate moieties with the N-termini of two PeptideY-Q's in which both Q's are Cam-esters, then the peptide coupling molecule may be assembled by i) preparing a first side-chain protected PeptideY-Cam-ester via solid-phase peptide synthesis and cleaving it from the resin; ii) preparing a second side-chain protected PeptideY-Cam-ester on the solid phase, reacting it with an excess of the compound containing two chloroformate groups, washing away the remaining

compound containing two chloroformate groups, cleaving the resulting mono-chloroformate mono-carbamate coupled side-chain protected PeptideY-Cam-ester from the resin; iii) coupling both PeptideY-Cam-esters by a reaction of the N-terminus of the first PeptideY-Cam-ester with the mono-chloroformate functionality of the second PeptideY-Cam-ester; iv) cleaving the side-chain protecting groups, giving the peptide coupling molecule based on two carbamate moieties.

For instance, when L is provided by two cysteines which are coupled via a disulfide bridge, then the peptide coupling molecule may be assembled by i) preparing a first PeptideY-Cam-ester, containing a Cys residue at position P5 or more remote from the Cam-ester end, via solid-phase peptide synthesis and cleaving it from the resin; ii) preparing a second PeptideY-Cam-ester, containing a Cys(SPy) residue at position P5 or more remote from the Cam-ester end, on the solid phase, and cleaving it from the resin (with the SPy group not being cleaved from the Cys residue); iii) coupling both PeptideY-Cam-esters by a reaction of the Cys residue of the first fragment with the Cys(SPy) residue of the second fragment giving the peptide coupling molecule based on a disulfide bridge.

For instance, when L is linked to 3 or more PeptideY-Q's, the synthesis of the peptide coupling molecule may require the use of protecting groups on the linking moiety. For instance, when L is provided by a tricarboxylic acid, the tricarboxylic acid may have two of the carboxylic acid residues protected with a protecting group which is not removed during the formation of amide bonds or during peptide cleavage. The peptide coupling molecule may then be assembled by i) preparing a first side-chain protected PeptideY-Cam-ester via solid-phase peptide synthesis and cleaving it from the resin and preparing a third side-chain protected PeptideY-Cam-ester via solid-phase peptide synthesis and cleaving it from the resin; ii) preparing a second side-chain protected PeptideY-Cam-ester on the solid phase, reacting it with an excess of the tricarboxylic acid which is protected on two of its carboxylic residues and standard peptide coupling reagents such as DCC or HBTU/Oxyma, washing away the excess tricarboxylic acid and cleaving the resulting second fragment from the resin giving a compound with one amide functionality between the triacid moiety and one PeptideY-Q; iii) selectively deprotecting one carboxylic acid residue of this second fragment; iv) coupling the first and second fragment (with one amide functionality) in the presence of standard peptide coupling reagents such as DCC or HBTU/Oxyma to give a compound in which two PeptideY-Q's are coupled to the triacid (via two amide bonds) and in which the third acid functionality is protected; iv) deprotecting the third acid functionality and coupling the resulting free carboxylic acid to the third peptide fragment using standard peptide coupling reagents such as DCC or HBTU/Oxyma; v) cleaving the side-chain protecting groups giving the peptide coupling molecule based on 3 amide groups.

A method for synthesizing a conjugate comprising enzymatically coupling a first nucleophile having an *N*-terminally unprotected amine to a first ester or thioester functionality Q-(C=O) of a peptide coupling molecule, and enzymatically coupling a second nucleophile having an *N*-terminally unprotected amine to a second ester or thioester functionality Q-(C=O) of the peptide coupling molecule according to the invention, can be carried out using ligases known per se to that purpose, variants thereof or homologues thereof, with the proviso that at least two different ligases are used. For example, one or more of the ligases may be selected from the group of serine-type carboxypeptidases, metallocarboxypeptidases, cysteine-type carboxypeptidases, serine endoproteases, aspartic endopeptidases, metalloendopeptidases, variants and homologues thereof.

Further, ligases can be designed based on, *e.g.*, the teaching of WO 2016/056913 or PCT/NL2016/050501.

In a preferred embodiment of a method according to the invention for synthesizing a conjugate, the first ligase is provided by a first variant of a serine endoprotease having a mutation of a serine in the hydrolytically active site of the serine endoprotease, which mutation is a substitution into cystein or selenocystein and/or the second ligase is provided by a second variant of a serine endoprotease having a mutation of a serine in the hydrolytically active site of the serine endoprotease, which mutation is a substitution into cystein or selenocystein.

Preferred ligases are selected from the group of subtilisins, subtilisin variants and homologues thereof. Particularly preferred are subtilisin BPN' variants or homologues thereof, having

- a deletion of the amino acids corresponding to positions L75, N76, N77, S78, 179, G80, V81, L82 and G83 of subtilisin BPN' (thus in general a deletion of a corresponding Ca2+ binding site);
- a mutation at the amino acid position corresponding to S221, the mutation being S221C or S221selenocysteine;
- preferably a mutation at the amino acid position corresponding to P225

wherein the amino acid positions are defined according to the sequence of subtilisin BPN' represented by SEQUENCE ID NO: 2.

Examples of suitable subtilisin BPN' variants are illustrated by SEQUENCE ID NO: 3 and SEQUENCE ID NO: 4. It should be noted that one or more further amino acid units may be mutated.

Such ligase has been found particularly suitable for synthesising a conjugate in a reaction medium comprising a substantial amount of water, in particular an aqueous reaction medium. Further, such ligase has been found particularly suitable not only for use as a catalyst in synthesizing a conjugate of relatively short oligopeptides, but also for synthesising a conjugate wherein at least one nucleophile is a relatively long peptide or a protein. Further, amongst these subtilisin BPN' variants or homologues thereof are ligases with a high selectivity towards coupling to a PeptideY, yet with a broad

spectrum activity towards coupling a peptide nucleophile.

A ligase used in accordance with the invention may have further mutations compared to subtilisin BPN', provided it has activity in the condensation of a PeptideY with a peptide nucleophile.

One or more further mutations in a ligase, in particular a subtilisin BPN' variant or homologue, are usually selected from

- mutations at the amino acid position corresponding to P225 of SEQUENCE ID NO: 2 selected from the group of P225N, P225D, P225S, P225C, P225G, P225A, P225T, P225V, P225I, P225L, P225H and P225Q;
- mutations selected from the group of mutations at an amino acid position corresponding to Q2, S3, P5, S9, I31, K43, M50, A73, E156, G166, G169, S188, Q206, N212, N218, T254 and Q271 of SEQUENCE ID NO 2, said one or more mutations preferably being selected from the group Q2K, S3C, P5S, S9A, I31L, K43N, M50F, A73L, E156S, G166S, G169A, S188P, Q206C, N212G, N218S, T254A and Q271E ;
- mutations at the amino acid position corresponding to S3C and Q206C (wherein the cysteins at the positions corresponding to position 3 and position 206 form a disulphide bridge) of SEQUENCE ID NO: 2;
- one or more mutations at the amino acid position corresponding to N62, G100, S125, L126, G127, P129, N155, Y217, N218 or M222 of SEQUENCE ID NO 2.
- at least one mutation selected from the group of mutations at an amino acid position corresponding to Y104, I107, L126, S101, G102, G127, G128, L135 and P168 of SEQUENCE ID NO 2.

Preferably the ligase comprises at least six, more preferably at least eight, more preferably 12-17 of said mutations selected from the group of Q2K, S3C, P5S, S9A, I31L, K43N, M50F, A73L, E156S, G166S, G169A, S188P, Q206C, N212G, N218S, T254A and Q271E of SEQUENCE ID NO 2.

A mutation at the position corresponding to M222 of SEQUENCE ID NO 2, preferably is M222G, M222P, M222N, M222E, M222Q or M222A, more preferably M222G or M222P.

A mutation at the amino acid position corresponding to Y217 of SEQUENCE ID NO 2, preferably is Y217L, Y217N, Y217E, Y217G, Y217F, Y217A, Y217S or Y217H.

A mutation at the position corresponding to Y104, I110 or I135 of SEQUENCE ID NO: 2 preferably is selected from the group of Y104F, Y104S, I107V, I107A, L135N, L135S, L135D and L135A.

Further details about mutations and how to obtain mutants with desired activities are described in WO 2016/056913 and PCT/NL2016/050501, of which the contents are incorporated by reference.

Alternatives to subtilisin BPN', as template enzymes from which an enzyme according to the invention, in particular a homologue of a subtilisin BPN' variant of the invention, can be derived by mutagenesis are other subtilisins, in particular subtilisins having at least 50 % homology with subtilisin BPN'.

Sequences of suitable subtilisins can be retrieved from the UNIPROT sequence database (http://www.uniprot.org/), as available on 11 August 2014, by BLASTing the database with subtilisin BPN' (SEQ ID 2) as a query. However sequence retrieval is not limited to UNIPROT nor to the date. The skilled person in the art knows how to query alternative sequence depositories or to collect additional homologue sequences by sequencing (see for example Zooming in on metagenomics: molecular microdiversity of Subtilisin Carlsberg in soil.,Gabor E, Niehaus F, Aehle W, Eck J.J Mol Biol. 2012 Apr 20;418(1-2):16-20).

One or more of the nucleophiles that are coupled are usually peptides, preferably each of the nucleophiles that are enzymatically coupled are peptides. As an alternative to a peptide, an aromatic amine may be coupled enzymatically using a method according to the invention.

In principle, a peptide coupling molecule, a first ligase, a second ligase (and optionally one or more further ligases) may be provided for synthesising a conjugate of any two or more peptide nucleophiles having an N-terminal free amine group.

The amino acid units of the peptide nucleophile may in principle be selected from any amino acid, proteinogenic or non-proteinogenic.

In particular, one or more peptide nucleophiles may be represented by a compound of the formula $H[-(NH)-(CR_AR_B)-(C=O)]_n-P^2$

Herein, n is an integer of at least 2, representing the number of amino acid units of the peptide nucleophile.

Each $R^A$ and each $R^B$ independently represent a hydrogen atom or an organic moiety, preferably an amino acid side-chain. Thus, it is not required that $R^A$ is the same in all n amino acid units. Similarly, it is not required that $R^B$ is the same in all n amino acid units. Optionally, one or more of the side-chain functionalities may contain a protecting group.

Herein $P^2$ represents an amine moiety or an OR moiety.

In case $P^2$ represents an amine moiety, the amine moiety may be represented by the formula $NR_3R_4$, in which $R_3$ and $R_4$ may each individually represent any (substituted or unsubstituted) alkyl or (substituted or unsubstituted) aryl group. In particular, one out of $R_3$ and $R_4$ is a hydrogen atom and the other a (substituted or unsubstituted) alkyl group.

Good results have particularly been obtained with $R_3$ and $R_4$ both being a hydrogen atom.

In case $P^2$ represents an OR moiety, R may represent a C-terminal protective group or a cation, for instance a monovalent cation, such as a tri- or tetrasubstituted ammonium ion or an alkaline metal cation or an H. In case R is a *C*-terminal protective group this may in particular be an optionally substituted alkyl group. Preferably it is a t-alkyl group, although in principle it also may be any other protective ester as known to a man skilled in the art. The t-alkyl may in principle be any protective tertiary alkyl group. Preferably the t-alkyl is selected from the group of t-butyl (2-methyl-2-propyl), t-pentyl (2-methyl-2-butyl) and t-hexyl (2,3-dimethyl-2-butyl).

In an embodiment, the (peptide nucleophile is *C*-terminal protected. In another embodiment it is not C-terminal protected.

The peptide nucleophile to be used in an enzymatic synthesis process of the invention may be synthesized using methods known in the art, such as solid-phase synthesis, solution phase synthesis or by fermentation using a microorganism. The N-terminal amino acid of the peptide nucleophile and the other amino acids of the peptide nucleophile may in principle be any amino acid, proteinogenic or non-proteinogenic.

The peptide nucleophile may be a linear peptide, a branched peptide or a peptide comprising a cyclic peptide structure. Generally, at least the N-terminal amino acid unit (P1') and the amino acid unit directly adjacent to the N-terminal amino acid unit (P2') are non-branched and not part of a cyclic structure. This is desired for facilitating enzymatic coupling of the nucleophile to the peptide coupling molecule. Preferably, in particular if a subtilisin BPN' variant or homologue thereof is used as a ligase, the P1' amino acid unit and the P2' amino acid unit are selected from the group of proteinogenic amino acid units other than proline and non-proteinogenic L-amino acid units, e.g. an L-isomer of a beta amino acid unit or an L-isomer of a gamma amino acid unit.

If the amino acid sequence of the N-terminal part of the peptide nucleophile is poorly recognized by or inaccessible to the ligase due to the amino acid preference of the ligase or due to the secondary or tertiary structure of the peptide nucleophile, the primary structure (amino acid sequence) of the peptide nucleophile may be elongated at the N-terminus. Essentially the N-terminus of the peptide nucleophile is elongated with a number of amino acids to ensure good recognition by and good accessibility to the ligase used for the enzymatic coupling reaction. The skilled person will know how to elongate the peptide nucleophile on the basis of the information disclosed herein and common general knowledge. Usually the number of amino acids for elongation is in the range of 1-10, although in principle it can be higher. Good result have been obtained by elongation of the peptide nucleophile with 3 amino acid residues, e.g. H-Ser-Tyr-Arg.

At least one of the nucleophiles preferably is an oligopeptide having 2-200 amino acid units, in particular having 5-100 amino acid units, more in particular 10-50 amino acid units.

In an advantageous embodiment, a first nucleophile is an oligopeptide, in particular a pharmaceutically active oligopeptide, and a second nucleophile is a protein.

The protein may be a protein improving target delivery (such as an antibody or antigen recognising fragment thereof having binding specificity to a specific organ tissue or cell) or a protein increasing the *in vivo* plasma half-time, *e.g.* albumin. In an embodiment, the protein is a hormone, such as insulin, or another pharmaceutically active protein.

It is also possible to provide a conjugate of a first and a second nucleophile that are both pharmaceutically active. Thus, the conjugate may be used for a combination therapy.

In an embodiment, one or more further nucleophiles are coupled to a conjugate of a first and a second nucleophile. E,g, a conjugate may be provided of one or more pharmaceutically active peptides, a peptide affecting the plasma-half time, and a peptide that improves target specificity.

Examples of pharmaceutically active peptides for use as a nucleophile in the synthesis of a conjugate according to the invention include Exenatide, Exenatide analogues (such as an analogue selected from the group of Glp-1, Teduglutide, Glucagon, Liraglutide and Semaglutide), Thymosin-alpha-1, Thymosin-alpha-1 analogues, Lixisenatide, Lixisenatide analogues, peptides comprising the sequence of any of these and one or more further amino acid units.

A conjugate comprising Exenatide, Lixisenatide or an analogue thereof can be used in the treatment of type 2 diabetes mellitus, in particular as adjunctive therapy to improve glycemic control in patients with type 2 diabetes mellitus who are taking metformin, but have not achieved adequate glycemic control.

A conjugate comprising Thymosin-alpha-1 or an analogue thereof can be used in a patient benefiting from enhancing cell-mediated immunity.

Examples of pharmaceutically active proteins include insulin receptor ligands, such as human insulin, porcine insulin, Humalog, aspart, insulin glulisine, insulin detemir, insulin degludec, and glargine insulin. A conjugate comprising an insulin receptor ligand is in particular useful for the treatment of diabetes.

The reaction conditions under which the synthesis of the peptide conjugate is carried out can be based on known conditions, depending on the ligases and nucleophiles used, the information disclosed herein, the literature cited herein and common general knowledge. Characteristic differences with known methods for enzymatic coupling of peptides reside in the use of a peptide linker molecule allowing coupling via the N-terminal amine function of each of the peptides and the use of two or more different ligases.

The coupling of two or more nucleophiles may be done in a single reaction medium comprising the peptide coupling molecule, each of the ligases, and each of the nucleophiles. Practically, it is preferred to carry out the coupling of the first nucleophile, the second nucleophile and optionally further nucleophiles in subsequent steps. This can be done by first adding the first ligase and the first nucleophile to a reaction medium, then allowing the first coupling to take place, thereafter adding the second ligase and the second nucleophile to the reaction medium, and then allowing the second coupling to take place. Thereafter, if desired one or more further nucleophiles may be coupled in an analogous matter. Alternatively in between subsequent coupling steps the intermediate product may be subjected to a recovery step or other processing step, after which the subsequent coupling may be carried out in a different reaction medium.

At least a first ligase to be used should have a higher selectivity towards coupling the first nucleophile to the first ester or thioester functionality Q-(C=O) over coupling the first nucleophile to the second ester or thioester functionality Q-(C=O). Preferably, the selectivity of a ligase towards the coupling reaction it is intended to catalyse is at least 5:1 in particular at least10:1, in particular at least 20:1. A higher selectivity of a ligase towards a specific coupling reaction of a specific nucleophile to a specific PeptideY does not require an upper limit; it may approach infinity. In practice, the selectivity of ligase towards a desired catalytic activity may be 100 000: 1 or less, 1 000:1 or less, 100:1 or less or 50:1 or less.

If the coupling of two or more different nucleophiles is done simultaneously in a single reaction medium, the first ligase should have a higher selectivity towards coupling the first nucleophile to the first ester or thioester functionality Q-(C=O) over coupling the first nucleophile to the second ester or thioester functionality Q-(C=O) (and preferably - if applicable - over any further ester or thioester functionality), preferably a selectivity of more than 5:1, more preferably of at least 10:1.

Further, in a coupling process wherein two or more different nucleophiles are coupled simultaneously in a single reaction medium, the second ligase should have a higher selectivity for coupling the second nucleophile to the second ester or thioester functionality Q-(C=O) over coupling the second nucleophile to the first ester or thioester functionality Q-(C=O) (and preferably - if applicable - over any further ester or thioester functionality), preferably a selectivity of more than 5:1, more preferably of at least 10:1. Any further ligase for coupling any further nucleophile should then likewise have a higher selectivity towards the coupling of said further nucleophile to a further PeptideY moiety.

If the synthesis process is carried out sequentially, a high selectivity is not needed for the last coupling step (the second coupling step, in case two nucleophiles are coupled).

In a method for synthesising a peptide conjugate according to the invention, at least when subtilisin variants or homologues thereof are used, the enzymatic coupling reaction is usually performed in a fluid comprising water. The reaction may be carried out in a fully aqueous liquid or in a mixture of water and a water miscible co-solvent such as N,N-dimethylformamide (DMF), N-methylpyrrolidinone (NMP), N,N-dimethylacetamide (DMA), dimethylsulphoxide (DMSO), acetonitrile, an ether, such as tetrahydrofuran (THF), 2-methyl-tetrahydrofuran (Me-THF) or 1,2-dimethoxyethane, or a (halogenated) alcohol, such as methanol, ethanol, isopropanol, tert-butanol, 2,2,2-trifluoroethanol (TFE), 1,1,1,3,3,3-hexafluoroisopropanol, or a mixture of these organic solvents. Depending on the stability of the ligase and the solubility of the peptide nucleophiles and the peptide coupling molecule, the amount of co-solvent is preferably below 70 vol%, more preferably below 60 vol%, even more preferably below 50 vol%, and most preferably below 40%. Provided the S/H ratio of the ligase so allows, the water content usually is 10-100 vol %, based on total liquids, preferably 20 vol. % or more, preferably 40 vol. % or more, in particular 50 vol. % or more, more in particular 60 vol. % or more, 80 vol.% or more or 95 vol. % or more. Subtilisin BPN' variants or homologues thereof, as referred to herein are in particular suitable for enzymatic coupling in a substantially or fully aqueous reaction medium,

Preferably the reaction is performed in a buffered fluid. In principle, any buffer is suitable. Good buffers are known to a person skilled in the art. See for instance David Sheehan in Physical Biochemistry, 2nd Ed. Wiley-VCH Verlag GmbH, Weinheim 2009; ; or http://www.amazon.com/Buffer-Solutions-BASICS-Garland-Science/dp/0199634424).

The pH of the buffer for enzymatic coupling may be at least 5, in particular at least 6, preferably at least 7. A desired maximum pH is usually less than 11, in particular less than 10, even more preferably less than 9. Usually the optimal pH for the enzymatic reactions is between 7 and 9.

In the coupling reaction with a ligase in a method according to the invention, a large excess of the peptide coupling molecule (the C-terminal ester or thioester) or of the peptide nucleophiles is generally not needed to reach a high yield in the condensation reaction. Usually the ratio of (a) the peptide coupling molecule to (b) the peptide nucleophiles is between 1:5 and 5:1, preferably in the range of 1.0:1.0 to 3:1, more preferably in the range of 1.1:1.0 to 2:1.

In particular, in a method of the invention for synthesising a peptide conjugate, it may be advantageous to add additives to the fluid wherein the reaction is carried out to improve the solubility of the peptide linking molecule or one or more nucleophiles or to improve the reaction yield. Such additives may be a salt or an organic molecule, for instance guanidinium hydrochloride, urea, sodium dodecasulphate or a polysorbate, such as Tween®.

In principle the temperature during the enzymatic coupling is not critical, as long as a temperature is chosen at which the ligase used shows sufficient activity and stability. Such a temperature is usually known for the ligase to be

used or can be routinely determined based on common general knowledge, the information disclosed herein and optionally a limited amount of testing. Generally, the temperature may be at least -10°C, in particular at least 0 °C or at least 10°C. Generally, the temperature may be 70°C or less, in particular 60°C or less or 50 °C or less. Optimal temperature conditions can easily be identified for a specific ligase for a specific enzymatic coupling by a person skilled in the art through routine experimentation based on common general knowledge and the information disclosed herein. In general, the temperature advantageously is in the range of 20-50°C.

An embodiment of the invention provides a peptide coupling reagent product, comprising a peptide coupling molecule according to the invention, a first ligase and a second ligase. Such product provides at least said components suitable for carrying out a synthesis according to the invention. The components can each independently be in a solid form (e.g. powder form) or in a liquid form. The ligases can be immobilized on a carrier. The peptide coupling reagent product can be a kit of parts, comprising the peptide coupling molecule, a first ligase and a second ligase each packaged in separate containers; it is also possible to combine the peptide coupling molecule and a first ligase in a first container and a second ligase in a second container, or to provide each of the peptide coupling molecule, a first ligase and a second ligase in the same container. The peptide coupling reagent product can comprise one or more further ligases, which may be in separate containers or packages in the same container as one or more of the other components. The peptide coupling reagent product may e.g. be offered for sale to designer labs or manufacturers of peptide conjugates, e.g. pharmaceutical companies, who can design respectively manufacture peptide conjugates for a specific use, e.g. a pharmaceutical use.

The invention will now be illustrated by the following examples.

**Example 1: synthesis of a peptide coupling molecule**

**Synthesis of side-chain protected fragment 1 (TFA.H-Phe-Ser(*t*Bu)-Lys(Boc)-Lys(Boc)-OCam-Leu-$NH_2$):**

TFA.H-Phe-Ser(*t*Bu)-Lys(Boc)-Lys(Boc)-OCam-Leu-$NH_2$ was synthesized on 1 gram of Fmoc-Leu-Sieber amide resin (loading of 0.7 mmol/gram) using standard solid phase peptide synthesis protocols.[1] For each coupling cycle, 4 eq of Fmoc-amino acid were used combined with HBTU/OxymaPure (4 eq) as coupling reagents in the presence of Di-isopropylethylamine (DiPEA, 8 eq). The Cam-ester was introduced as described in the literature[2] using an Fmoc-Lys(Boc)-$OCH_2COOH$ building block. The peptide was cleaved using 2 vol% trifluoroacetic acid (TFA) in dichloromethane (DCM) (15 min) followed by filtration (both cleavage and filtration 3x). The combined organic fractions were concentrated *in vacuo* and the crude product was purified by preparative HPLC. The gradient was 100% buffer A (5 vol% acetonitrile and 0.05 vol% TFA in water) to 100% buffer B (5 vol% water and 0.05 vol% TFA in acetonitrile) during 30 min. Pure fractions were combined and lyophilized.

**Synthesis of side-chain protected fragment 2 (HO-(C=O)-$CH_2$-$CH_2$-(C=O)-Phe-Ser(*t*Bu)-Lys(Boc)-Glu(O*t*-Bu)-OCam-Leu-$NH_2$):**

HO-(C=O)-$CH_2$-$CH_2$-(C=O)-Phe-Ser(*t*Bu)-Lys(Boc)-Glu(O*t*Bu)-OCam-Leu-$NH_2$ was synthesized on 1 gram of Fmoc-Leu-Sieber amide resin (loading of 0.7 mmol/gram) using standard solid phase peptide synthesis protocols.[1] For each coupling cycle, 4 eq of Fmoc-amino acid were used combined with HBTU/OxymaPure (4 eq) as coupling reagents in the presence of DiPEA (8 eq). The Cam-ester was introduced as described in the literature[2] using an Fmoc-Glu(O*t*-Bu)-$OCH_2COOH$ building block. The HO-(C=O)-$CH_2$-$CH_2$-(C=O)- moiety was introduced using succinic anhydride (4 eq) and DiPEA (8 eq). The peptide was cleaved using 2 vol% TFA in DCM (15 min) followed by filtration (both cleavage and filtration 3x). The combined organic fractions were concentrated *in vacuo* and the crude product was purified by preparative HPLC. The gradient was 100% buffer A (5 vol% acetonitrile and 0.05 vol% TFA in water) to 100% buffer B (5 vol% water and 0.05 vol% TFA in acetonitrile) during 30 min. Pure fractions were combined and lyophilized.

**Condensation of protected fragments 1 and 2, and deprotection, yielding the peptide coupling molecule:**

┌ $CH_2$-(C=O)-Phe-Ser-Lys-Glu-OCam-Leu-$NH_2$.3TFA |
└ $CH_2$-(C=O)-Phe-Ser-Lys-Lys-OCam-Leu-$NH_2$

100 mg of HO-(C=O)-$CH_2$-$CH_2$-(C=O)-Phe-Ser(*t*Bu)-Lys(Boc)-Glu(O*t*Bu)-OCam-Leu-$NH_2$ (fragment 2) was

dissolved in 5 mL DMF and HBTU/Oxyma was added (2 eq) followed by the addition of DiPEA (4 eq). After stirring for 10 min, 100 mg of TFA.H-Phe-Ser($^{t}$Bu)-Lys(Boc)-Lys(Boc)-OCam-Leu-$NH_2$ (fragment 1) was added. The reaction mixture was stirred overnight. The crude product was precipitated in water, isolated by filtration and dried by lyophilization. The peptide was side-chain deprotected using TFA/TIS/water (95/2.5/2.5, v/v/v) for 120 min and precipitated in MTBE. The precipitated peptide was purified by preparative HPLC. The gradient was 100% buffer A (5 vol% acetonitrile and 0.05 vol% TFA in water) to 100% buffer B (5 vol% water and 0.05 vol% TFA in acetonitrile) during 30 min. Pure fractions were combined and lyophilized. The peptide coupling molecule product was obtained in 10% yield and 97% purity.

LITERATURE:

1) Fmoc Solid Phase Peptide Synthesis: A Practical Approach, W. Chan, Peter White, Oxford University Press, Oxford, 2000**.**

2) Improved solid phase synthesis of peptide carboxyamidomethyl (Cam) esters for enzymatic segment condensation, T. Nuijens et al., Tetrahedron Letters 2016, 57(32), 3635-3638.


**Example 2: synthesis of a conjugate using the peptide coupling molecule from Example 1**

2 Mg of peptide coupling molecule as obtained by the method as described in Example 1 was dissolved in 1 mL of phosphate buffer (1 N, pH 8.3). To this solution, 1 mg of tris(2-carboxyethyl)phosphine(TCEP), 1 mg of H-Ala-Leu-Arg-$NH_2$.2TFA and 5 μg of ligase-1 was added, which resulted in the selective coupling of the tripeptide to the upper PeptideY moiety of the peptide coupling molecule. The main product identified by LC-MS after 30 minutes stirring was:

┌ $CH_2$-(C=O)-Phe-Ser-Lys-Glu-Ala-Leu-Arg-$NH_2$

└ $CH_2$-(C=O)-Phe-Ser-Lys-Lys-OCam-Leu-$NH_2$

Subsequently, 5 mg of H-Glu-Leu-Arg-$NH_2$.2TFA and 5 μg of ligase-2 were added which resulted in the selective coupling of the tripeptide to the lower PeptideY moiety of the peptide coupling molecule. The main product identified by LC-MS after another 30 minutes stirring was:

┌ $CH_2$-(C=O)-Phe-Ser-Lys-Glu-Ala-Leu-Arg-$NH_2$

└ $CH_2$-(C=O)-Phe-Ser-Lys-Lys-Glu-Leu-Arg-$NH_2$

**SEQUENCES**

SEQ ID NO 1: wild type gene encoding for subtilisin BPN' amino acids -107 to 275 ENA |K02496| K02496.1 B. Subtilisin BPN' Bacillus amyloliquefaciens

GTGAGAGGCAAAAAAGTATGGATCAGTTTGCTGTTTGCTTTAGCGTTAATCTTTAC GATGG

CGTTCGGCAGCACATCCTCTGCCCAGGCGGCAGGGAAATCAAACGGGGAAAAGAA ATATAT

TGTCGGGTTTAAACAGACAATGAGCACGATGAGCGCCGCTAAGAAGAAAGATGTC ATTTCT

GAAAAAGGCGGGAAAGTGCAAAAGCAATTCAAATATGTAGACGCAGCTTCAGCTA CATTAA

ACGAAAAAGCTGTAAAAGAATTGAAAAAAGACCCGAGCGTCGCTTACGTTGAAGA AGATCA

CGTAGCACATGCGTACGCGCAGTCCGTGCCTTACGGCGTATCACAAATTAAAGCC CCTGCT

CTGCACTCTCAAGGCTACACTGGATCAAATGTTAAAGTAGCGGTTATCGACAGCG GTATCG

ATTCTTCTCATCCTGATTTAAAGGTAGCAGGCGGAGCCAGCATGGTTCCTTCTGAA ACAAA

TCCTTTCCAAGACAACAACTCTCACGGAACTCACGTTGCCGGCACAGTTGCGGCTC TTAAT

AACTCAATCGGTGTATTAGGCGTTGCGCCAAGCGCATCACTTTACGCTGTAAAAGT TCTCG

GTGCTGACGGTTCCGGCCAATACAGCTGGATCATTAACGGAATCGAGTGGGCGAT CGCAAA

CAATATGGACGTTATTAACATGAGCCTCGGCGGACCTTCTGGTTCTGCTGCTTTAA AAGCG

GCAGTTGATAAAGCCGTTGCATCCGGCGTCGTAGTCGTTGCGGCAGCCGGTAACG AAGGCA

CTTCCGGCAGCTCAAGCACAGTGGGCTACCCTGGTAAATACCCTTCTGTCATTGCA GTAGG

CGCTGTTGACAGCAGCAACCAAAGAGCATCTTTCTCAAGCGTAGGACCTGAGCTT
GATGTC
ATGGCACCTGGCGTATCTATCCAAAGCACGCTTCCTGGAAACAAATACGGGGCGT
ACAACG
GTACGTCAATGGCATCTCCGCACGTTGCCGGAGCGGCTGCTTTGATTCTTTCTAAG
CACCC
GAACTGGACAAACACTCAAGTCCGCAGCAGTTTAGAAAACACCACTACAAAACTTG
GTGAT
TCTTTCTACTATGGAAAAGGGCTGATCAACGTACAGGCGGCAGCTCAGTAA

SEQ ID NO 2: wild type subtilisin BPN' (mature)
>SUBT_BACAM Subtilisin BPN' Bacillus amyloliquefaciens mature 1 to 275

AQSVPYGVSQIKAPALHSQGYTGSNVKVAVIDSGIDSSHPDLKVAGGASMVPSETNP
FQDNNSHGTHVAGTVAALNNSIGVLGVAPSASLYAVKVLGADGSGQYSWIINGIEW
AIANNMDVINMSLGGPSGSAALKAAVDKAVASGVVVVAAAGNEGTSGSSSTVGYPG
KYPSVIAVGAVDSSNQRASFSSVGPELDVMAPGVSIQSTLPGNKYGAYNGTSMASP
HVAGAAALILSKHPNWTNTQVRSSLENTTTKLGDSFYYGKGLINVQAAAQ

SEQ ID NO 3: subtilisin BPN' variant with deletion of $Ca^{2+}$ binding loop and S221C and preferably P225 mutation (denoted as P225X)

AQSVPYGVSQIKAPALHSQGYTGSNVKVAVIDSGIDSSHPDLKVAGGASMVPSETNP
FQDNNSHGTHVAGTVAAVAPSASLYAVKVLGADGSGQYSWIINGIEWAIANNMDVI
NMSLGGPSGSAALKAAVDKAVASGVVVVAAAGNEGTSGSSSTVGYPGKYPSVIAVG
AVDSSNQRASFSSVGPELDVMAPGVSIQSTLPGNKYGAYNGTCMASXHVAGAAALI
LSKHPNWTNTQVRSSLENTTTKLGDSFYYGKGLINVQAAAQ

SEQ ID NO 4: subtilisin BPN' variant with preferred mutation positions compared to SEQ ID NO 3

AXXVXYGVXQIKAPALHSQGYTGSNVKVAVXDSGIDSSHPDLXVAGGASXVPSETNP
FQDNNSHGTHVAGTVXAVAPSASLYAVKVLGADGSGQYSWIINGIEWAIANNMDVI

NMSLGGPSGSAALKAAVDKAVASGVVVVAAAGNXGTSGSSSTVXYPXKYPSVIAVG
AVDSSNQRAXFSSVGPELDVMAPGVSIXSTLPGXKYGAXXGTCXASXHVAGAAALIL
SKHPNWTNTQVRSSLENTXTKLGDSFYYGKGLINVXAAAQ

## Claims

1. Peptide coupling molecule represented by the formula

L{- PeptideY-Q}$_x$

wherein
x is an integer having a value of at least 2;
L is a linker moiety;
each PeptideY independently represents a peptide chain comprising at least four amino acid units, each PeptideY having a terminal carbonyl group (C=O)
each Q independently represents a functional group forming an ester or thioester functionality with the terminal carbonyl group of the PeptideY to which Q is bound; and
at least two PeptideY's of the peptide coupling molecule are different from each other.

**2.** Peptide coupling molecule according to claim 1, wherein at least one Q, preferably each Q, - represents a formula $-OCX_2$-Z or a formula $-SCX_2$-Z, wherein each X independently represents a hydrogen atom, a halogen atom, a substituted or unsubstituted alkyl group or a substituted or unsubstituted aryl group
and Z is selected from the group of $sp^3$-hybridized carbons comprising at least two substituents comprising a heteroatom directly attached to the $sp^3$-hybridized carbon and $sp^2$ hybridized carbons comprising one or two substituents comprising a heteroatom directly attached to the $sp^2$-hybridized carbon, Z preferably being selected from the group consisting of carbamoyl groups, trifluoroalkyl groups, trichloroalkyl groups, and cyanoalkyl groups.

**3.** Peptide coupling molecule according to claim 2, wherein at least one Z, preferably each Z, is a carbamoyl group represented by the formula -C(=O)($NR^1R^2$) wherein;
$R_1$ represents a hydrogen atom or a substituted or unsubstituted alkyl group or a substituted or unsubstituted aryl group; and
$R^2$ represents a hydrogen atom or a substituted or unsubstituted alkyl group or a substituted or unsubstituted aryl group or an amino acid or peptide residue with a C-terminal carboxyamide or carboxylic acid functionality, optionally protected on the side-chain functionality of the amino acid residue or on one or more of the side-chain functionalities of the peptide residue

**4.** Peptide coupling molecule according to claim 3, wherein one or more Q's, preferably each Q,, represents the formula $-O-CH_2-C(=O)(NHR^2)$, wherein $R^2$ represents a hydrogen atom or an amino acid or peptide residue with a C-terminal carboxyamide or carboxylic acid functionality.

**5.** Peptide coupling molecule according to any of the preceding claims, wherein L is

- an organic moiety having two or more carbonyl groups, in particular a dicarbonyl moiety having the formula $(O=C)(CH_2)_n$ (C=O) with n=1-14, a dicarbonyl moiety having the formula $-(O=C)(C_nH_{2n-2p})$ (C=O)- with n=2-14 and p being an integer indicating the number of unsaturations, a tricarbonyl moiety having the formula $(C_n H_{2n-1})$ $(C=O)_3$ with n=3-14 or a tricarbonyl moiety having the formula $(C_n H_{2n-1-2p})$ $(C=O)_3$ with n=4-14 and p being an integer indicating the number of unsaturations (e.g. a succinyl, glutaryl, sebacoyl, a maleoyl or citroyl group), two or more of said carbonyl groups forming an amide with the N-terminal amine functionality of a PeptideY; or
- an amino acid residue of an amino acid having at least two carboxylic acid functionalities (e.g. aspartic acid or glutamic acid) said amino acid residue comprising two carbonyl groups forming an amide with the N-terminal amine functionality of a PeptideY ; or
- a peptide chain bound to the N-terminal ends of each PeptideY (e.g. Lys-Asp or Lys-Glu).

**6.** Peptide coupling molecule according to any of the preceding claims, represented by the formula $Q_A[-(C=O)-(CR_AR_B)-(NH)-]_n-L -[(NH)-(CR_AR_B)-(C=O)-]_m-Q_B$ , wherein
$Q_A$ and $Q_B$ are as defined for Q in the preceding claims, preferably forming a carboxyamidomethyl-ester;
n is an integer having a value in the range of 4-20, preferably in the range of 4-10, more preferably, 4, 5, 6 or 7;
m is in integer having a value in the range of 4-20, preferably in the range of 4-10, more preferably, 4, 5, 6 or 7;
and each $R_A$ and each $R_B$ independently represent a hydrogen atom or an amino acid side-chain of a proteinogenic amino acid.

**7.** A method for synthesizing a conjugate comprising enzymatically coupling a first nucleophile having an N-terminally unprotected amine - preferably a first peptide having an N-terminally unprotected amine - to a first ester or thioester functionality Q-(C=O) of a peptide coupling molecule according to any of the preceding claims, which coupling is catalysed by a first ligase and
enzymatically coupling a second nucleophile, having an N-terminally unprotected amine - preferably a second

peptide, different from the first peptide, having an *N*-terminally unprotected amine - to a second ester or thioester functionality Q-(C=O) of said peptide coupling molecule, which coupling is catalysed by a second ligase.

**8.** Method according to claim 7, wherein the first ligase is a first variant of a serine endoprotease having a mutation of the serine residue in the hydrolytically active site, which mutation is a substitution into cystein or selenocystein and wherein the second ligase is a second variant of a serine endoprotease having a mutation of the serine residue in the hydrolytically active site , which mutation is a substitution into cystein or selenocystein.

**9.** Method according to claim 8, wherein said first ligase and/or said second ligase is a subtilisin BPN' variant or a homologue thereof, which comprises the following mutations compared to subtilisin BPN' represented by SEQUENCE ID NO: 2 or a homologue sequence thereof :

- a deletion of the amino acids corresponding to positions 75-83;
- a mutation at the amino acid position corresponding to S221, the mutation being S221C or S221selenocysteine;
- preferably a mutation at the amino acid position corresponding to P225;

wherein the amino acid positions are defined according to the sequence of subtilisin BPN' represented by SEQUENCE ID NO: 2.

**10.** Method according to claim 7, 8 or 9, wherein one or more of the enzymatic coupling reactions, preferably each of the enzymatic coupling reactions of a nucleophile having an N-terminally unprotected amine to the peptide coupling molecule is carried out in a reaction medium, comprising at least 50 vol % water, preferably at least 60 vol % water.

**11.** Method according to any of the claims 7-10, wherein the first nucleophile is a peptide, preferably a peptide having 2-200 amino acid units, in particular having 5-100 amino acid units, more in particular 10-50 amino acid units and/or wherein the second nucleophile is a protein.

**12.** Method according to any of the claims 7-11, wherein the selectivity of the first ligase for coupling the first nucleophile to the first ester or thioester functionality Q-(C=O) over coupling the first nucleophile to the second ester or thioester functionality Q-(C=O) is at least 10:1 and/or
wherein the selectivity of the second ligase for coupling the second nucleophile to the second ester or thioester functionality Q-(C=O) over coupling the second nucleophile to the first ester or thioester functionality Q-(C=O) is at least 10:1.

**13.** Peptide conjugate represented by the formula

$$L\{\text{-PeptideY-(C=O)-PeptideZ}\}_x$$

wherein
x, PeptideY and L are as defined in any of the preceding claims, and wherein
each PeptideZ is a peptide bound to -(C=O) - PeptideY via its N-terminal amino acid residue.

**14.** Peptide coupling reagent product, comprising a peptide coupling molecule according to any of the claims 1-6, a first ligase and a second ligase, which first and second ligase are preferably as defined in claim 8 or 9, preferably a peptide coupling reagent product selected from the group of

- kits of parts comprising a first container containing the peptide coupling molecule, a second container comprising the first ligase and a third container comprising the second ligase;
- kits of parts comprising a first container containing the peptide coupling molecule and a second container comprising the first ligase and the second ligase; and
- peptide coupling reagent products comprising a mixture, the mixture comprising the peptide coupling molecule, the first ligase and the second ligase.

**15.** Peptide conjugate according to claim 13, comprising at least one pharmaceutically active PeptideZ for use as a medicament.

Europäisches Patentamt
European Patent Office
Office européen des brevets

# EUROPEAN SEARCH REPORT

Application Number
EP 16 19 0641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHENG DING ET AL: "Multivalent Antiviral XTEN-Peptide Conjugates with Long in Vivo Half-Life and Enhanced Solubility", BIOCONJUGATE CHEMISTRY, vol. 25, no. 7, 16 July 2014 (2014-07-16), pages 1351-1359, XP055354811, ISSN: 1043-1802, DOI: 10.1021/bc500215m * the whole document * ----- | 1-15 | INV. B01J19/00 |
| A | WO 2016/056913 A1 (ENZYPEP B V [NL]; UNIV GRONINGEN [NL]) 14 April 2016 (2016-04-14) * page 19, line 33 - page 21, line 31; claims 1-76 * ----- | 1-15 | |
| A | CHRISTIAN P. R. HACKENBERGER ET AL: "Chemoselective Ligation and Modification Strategies for Peptides and Proteins", ANGEWANDTE CHEMIE INTERNATIONAL EDITION, vol. 47, no. 52, 15 December 2008 (2008-12-15), pages 10030-10074, XP055087404, ISSN: 1433-7851, DOI: 10.1002/anie.200801313 * Scheme 31B * ----- | 1-15 | TECHNICAL FIELDS SEARCHED (IPC) B01J |
| A | DE BEER R J A C ET AL: "Improving the carboxyamidomethyl ester for subtilisin A-catalysed peptide synthesis", ORGANIC AND BIOMOLECULAR CHEMISTRY 20120907 ROYAL SOCIETY OF CHEMISTRY GBR, vol. 10, no. 33, 7 September 2012 (2012-09-07), pages 6767-6775, XP029641152, DOI: 10.1039/C2OB25662B * abstract * * Scheme 1; table 1 * ----- | 1-15 | |

-/--

The present search report has been drawn up for all claims

3

| Place of search | Date of completion of the search | Examiner |
|---|---|---|
| Munich | 20 March 2017 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 0641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BONDALAPATI SOMASEKHAR ET AL: "Expanding the chemical toolbox for the synthesis of large and uniquely modified proteins", NATURE CHEMISTRY, vol. 8, no. 5, May 2016 (2016-05), pages 407-418, XP002768310, * abstract; figure 2 * ----- | 1-15 | |
| A | GUAN XIAOYANG ET AL: "New Methods for Chemical Protein Synthesis", PROTEIN LIGATION AND TOTAL SYNTHESIS II SPRINGER-VERLAG BERLIN, HEIDELBERGER PLATZ 3, D-14197 BERLIN, GERMANY SERIES : TOPICS IN CURRENT CHEMISTRY (ISSN 0340-1022(PRINT)), 2015, pages 155-192, XP009193735, * page 177, paragraph 3.2.1 - page 181, paragraph 1 * ----- | 1-15 | |
| A | RAIBAUT LAURENT ET AL: "Solid Phase Protein Chemical Synthesis", PROTEIN LIGATION AND TOTAL SYNTHESIS II SPRINGER-VERLAG BERLIN, HEIDELBERGER PLATZ 3, D-14197 BERLIN, GERMANY SERIES : TOPICS IN CURRENT CHEMISTRY (ISSN 0340-1022(PRINT)), 2015, pages 103-154, XP009193736, * the whole document * ----- | 1-15 | TECHNICAL FIELDS SEARCHED (IPC) |

The present search report has been drawn up for all claims

3

| Place of search | Date of completion of the search | Examiner |
|---|---|---|
| Munich | 20 March 2017 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 16 19 0641

This annex lists the patent family members relating to the patent documents cited in the above-mentioned European search report. The members are as contained in the European Patent Office EDP file on
The European Patent Office is in no way liable for these particulars which are merely given for the purpose of information.

20-03-2017

| Patent document cited in search report | Publication date | Patent family member(s) | Publication date |
| --- | --- | --- | --- |
| WO 2016056913 A1 | 14-04-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5403737 A
- WO 2016056913 A
- WO 2010057961 A
- NL 2016050501 W


### Non-patent literature cited in the description


- **SAMBROOK et al.** Molecular Cloning-A Laboratory Manual. 1989, vol. 1-3
- **SCHECHTER ; BERGER.** *Biochem Biophys Res Commun.,* 20 April 1967, vol. 27 (2), 157-62
- Time warps, string edits and macromolecules: the theory and practice of sequence comparison. **KRUSKAL, J. B.** An overview of sequence comparison In. Addison Wesley, 1983, 1-44
- **NEEDLEMAN, S. B. ; WUNSCH, C. D.** *J. Mol. Biol.,* 1970, vol. 48, 443-453
- **RICE,P. ; LONGDEN,I. ; BLEASBY,A.** EMBOSS: The European Molecular Biology Open Software Suite. *Trends in Genetics,* 2000, vol. 16 (6), 276-277, http://emboss.bioinformatics.nl/
- **GABOR E ; NIEHAUS F ; AEHLE W ; ECK J.** Zooming in on metagenomics: molecular microdiversity of Subtilisin Carlsberg in soil. *J Mol Biol.,* 20 April 2012, vol. 418 (1-2), 16-20
- David Sheehan in Physical Biochemistry. Wiley-VCH Verlag GmbH, 2009
- **W. CHAN ; PETER WHITE.** Fmoc Solid Phase Peptide Synthesis: A Practical Approach. Oxford University Press, 2000
- **T. NUIJENS et al.** Improved solid phase synthesis of peptide carboxyamidomethyl (Cam) esters for enzymatic segment condensation. *Tetrahedron Letters,* 2016, vol. 57 (32), 3635-3638